# EUROPÄISCHE PATENTSCHRIFT

(11) **EP 2 360 250 B1**
(45) Veröffentlichungstag und Bekanntmachung des Hinweises auf die Patenterteilung: **04.03.2015**
(21) Anmeldenummer: 10180546.3
(22) Anmeldetag: 19.01.2006
(51) Int. Cl.: C12N 15/11, A61K 48/00

(54) **siRNA-Moleküle zur Behandlung von Blutgefäßen**
siRNA for treatment of blood vessels
MOLECULES DE ARNSI POUR LE TRAITEMENT DE VAISSEAUX SANGUINS

(30) Priorität: 19.01.2005 DE 102005003788
(43) Veröffentlichungstag der Anmeldung: 24.08.2011
(62) Teilanmeldung aus: 06702980.1
(73) Patentinhaber: Eberhard-Karls-Universität Tübingen Universitätsklinikum, 72076 Tübingen (DE)
(72) Erfinder: Walker, Tobias, 72074, Tübingen (DE); Wendel, Hans-Peter, 72336, Balingen (DE)
(74) Vertreter: Witte, Weller & Partner Patentanwälte mbB

(56) Entgegenhaltungen:
- WO-A-03/104456
- WO-A2-2004/030634
- WO-A2-2004/065546
- POSTON ET AL: "Ex vivo gene therapy prevents chronic graft vascular disease in cardiac allografts", JOURNAL OF THORACIC AND CARDIOVASCULAR SURGERY, MOSBY-YEAR BOOK, INC., ST. LOUIS, MO, US, Bd. 116, Nr. 3, 1. September 1998 (1998-09-01), Seiten 386-396, XP005155585, ISSN: 0022-5223, DOI: DOI:10.1016/S0022-5223(98)70003-0
- B FEELEY: "Pressure delivery of AS-ICAM-1 ODN with LFA-1 mAb reduces reperfusion injury in cardiac allografts", THE ANNALS OF THORACIC SURGERY, Bd. 68, Nr. 1, 1. Juli 1999 (1999-07-01), Seiten 119-124, XP55000166, ISSN: 0003-4975, DOI: 10.1016/S0003-4975(99)00503-2
- POSTON ROBERT S ET AL: "Antisense oligodeoxynucleotides prevent acute cardiac allograft rejection via a novel, nontoxic, highly efficient transfection method", TRANSPLANTATION, WILLIAMS AND WILKINS, BALTIMORE US, Bd. 68, Nr. 6, 27. September 1999 (1999-09-27), Seiten 825-832, XP009149049, ISSN: 0041-1337
- FEELEY BRIAN T ET AL: "Optimization of ex vivo pressure mediated delivery of antisense oligodeoxynucleotides to ICAM-1 reduces reperfusion injury in rat cardiac allografts", TRANSPLANTATION, WILLIAMS AND WILKINS, BALTIMORE US, Bd. 69, Nr. 6, 27. März 2000 (2000-03-27), Seiten 1067-1074, XP009149048, ISSN: 0041-1337
- HEIN M ET AL: "GENE TRANSFER INTO RAT HEART-DERIVED ENDOTHELIAL CELLS", EUROPEAN JOURNAL OF CARDIO-THORACIC SURGERY, SPRINGER VERLAG, BERLIN, DE, Bd. 13, Nr. 4, 1. März 1998 (1998-03-01), Seiten 460-466, XP000876917, ISSN: 1010-7940, DOI: DOI:10.1016/S1010-7940(98)00029-3
- KATIRCIOGLU S F ET AL: "Iloprost Added to the Cardioplegic Solutions Improves Myocardial Performance - A potentially valuable agent for preserving myocardial tissue in acute myocardial ischaemia", PROSTAGLANDINS, BUTTERWORTH, STONEHAM, MA, US, Bd. 55, Nr. 1, 1. Januar 1998 (1998-01-01) , Seiten 51-65, XP004113179, ISSN: 0090-6980, DOI: DOI:10.1016/S0090-6980(98)00006-9
- NISHIWAKI Y ET AL: "Introduction of short interfering RNA to silence endogenous E-selectin in vascular endothelium leads to successful inhibition of leukocyte adhesion", BIOCHEMICAL AND BIOPHYSICAL RESEARCH COMMUNICATIONS, ACADEMIC PRESS INC. ORLANDO, FL, US, Bd. 310, Nr. 4, 31. Oktober 2003 (2003-10-31), Seiten 1062-1066, XP004464071, ISSN: 0006-291X
- VASSALLI G ET AL: "Gene transfer of cytoprotective and immunomodulatory molecules for prevention of cardiac allograft rejection", EUROPEAN JOURNAL OF CARDIO-THORACIC SURGERY, SPRINGER VERLAG, BERLIN, DE, Bd. 24, 1. November 2003 (2003-11-01), Seiten 794-806, XP003005042, ISSN: 1010-7940, DOI: DOI:10.1016/S1010-7940(03)00456-1
- WALKER ET AL: "Suppression of ICAM-1 in human venous endothelial cells by small interfering RNAs", EUROPEAN JOURNAL OF CARDIO-THORACIC SURGERY, SPRINGER VERLAG, BERLIN, DE, Bd. 28, Nr. 6, Dezember 2005 (2005-12), Seiten 816-820, XP005230604, ISSN: 1010-7940
- YANG Q ET AL: "Effect of Cardioplegic and Organ Preservation Solutions and Their Components on Coronary Endothelium-Derived Relaxing Factors", THE ANNALS OF THORACIC SURGERY, ELSEVIER, Bd. 80, Nr. 2, 1. August 2005 (2005-08-01) , Seiten 757-767, XP004985894, ISSN: 0003-4975, DOI: DOI:10.1016/J.ATHORACSUR.2004.10.003

## Beschreibung

Die vorliegende Erfindung betrifft siRNA-Moleküle zur Inhibition der Expression von endothelialen Adhäsionsmolekülen.

In westlichen Industrieländern gehört die sog. koronare Herzkrankheit zu den häufigsten Todesursachen. Hierunter versteht man die durch verengte Herzkranzgefäße verursachte Mangeldurchblutung des Herzmuskels. Diese Mangeldurchblutung führt - vor allem unter Belastung - zu einem Missverhältnis von angebotenem und benötigtem Sauerstoff. Die wichtigste Ursache der koronaren Herzkrankheit ist die Arteriosklerose der Koronararterien, also der Herzkranzgefäße. Die Folgen können eine Angina Pectoris mit dadurch ausgelösten Brustschmerzen sowie ein Herzinfarkt sein.

Derzeit verwendete Arzneimittel gegen die koronare Herzkrankheit, sog. Koronartherapeutika, umfassen Nitrate, auch Nitroverbindungen genannt, Betablocker und Calciumantagonisten. Diese Substanzen bewirken, dass das Herz für seine Arbeit weniger Sauerstoff benötigt und dadurch entlastet wird. Der durch die koronare Herzkrankheit bedingte Sauerstoffmangel am Herzen wird teilweise ausgeglichen.

Nachteilig bei einer solchen medikamentösen Behandlung ist allerdings, dass insbesondere bei fortgeschrittener Krankheit häufig keine ausreichende Entlastung des Herzens mehr erzielt werden kann. Auch weisen eine Vielzahl der gängigen Arzneimittel gegen die koronare Herzkrankheit unerwünschte Nebenwirkungen auf.

Ein invasiver Ansatz zur Behandlung der koronaren Herzkrankheit besteht in der Durchführung einer Angioplastie. Bei einem solchen Verfahren wird in das verengte Koronargefäß ein Ballonkatheter eingeführt und durch Aufblasen des Letzteren wird versucht das Gefäßvolumen zu erweitern und die Gefäßinnenwand zu glätten. Eine solche Angioplastie wird üblicherweise mit der Einlagerung eines Stents kombiniert, um das verstopfte oder verengte Gefäß dauerhaft zu weiten.

Diese Maßnahme birgt jedoch für den betroffenen Patienten eine Vielzahl von Risiken, die in der Invasivität des Verfahrens begründet sind. Darüber hinaus ist dieses Verfahren bei sehr ausgeprägter Verkalkung der Stenose, Störung der Blutgerinnung oder ungünstiger anatomischer Lage des Gefäßes kontraindiziert. Ferner kann es zur Nachblutung, zur Thrombosierung, Embolie, Perforation, Dissektion etc. kommen. Da mit diesem Vorgehen kein dauerhafter Schutz vor einem erneuten Gefäßverschluss sichergestellt werden kann, ist es in vielen Fällen erforderlich, die angioplastischen Maßnahmen zu wiederholen. Dabei wird der Patient erneut den vorstehend genannten Gefahren ausgesetzt. Bei fortgeschrittener Erkrankung ist eine herzchirurgische Operation des Patienten unvermeidbar.

Ein weiterer therapeutischer Ansatz zur Behandlung der koronaren Herzkrankheit besteht in der Transplantation von Gefäßen, beispielsweise eines autologen Vena saphena-Segmentes in Form eines Bypasses. Mittels dieses Eingriffes wird versucht eine Stenose oder einen Totalverschluss einer oder mehrerer Koronararterien durch Einsetzen eines Transplantates zwischen der Aorta und der Koronararterie zu umgehen. Diese Operation stellt heute immer noch das Standardverfahren bei der invasiven Therapie der koronaren Herzkrankheit dar. Weltweit werden ca. 1 Million Patienten pro Jahr operiert, allein in Deutschland ca. 80.000.

Die verwendeten Bypass-Transplantate unterliegen intraoperativ einer Vielzahl von Stressfaktoren: mechanische Traumata, Streching, Hypoxie, Hypothermie, Cytokinstimulierung über die Herz-Lungen-Maschine, etc. Zudem wird nach erfolgreicher Revascularisierung das ursprünglich venöse Gefäß einem arteriellen Druck ausgesetzt, was in einer starken Belastung des Gefäßes resultiert.

All diese Faktoren führen zu einer fulminanten Endothelaktivierung, die eine gesteigerte über interzelluläre Adhäsionsmoleküle vermittelte transendotheliale Migration von Leucozyten hervorruft, welche massiv die Integrität des Endhothels beinträchtigt und somit wesentlich zur frühen Bypass-Stenose beiträgt; vgl. Chello et al. (2003), Pressure distension stimulates the expression of endothelial adhesion molecules in the human saphenous vein graft, Ann. Thorac. Surg. 76(2), S. 453-458. So beträgt die Offenheitsrate von Bypass-Transplantaten nach 10 Jahren bei Verwendung von arteriellen Gefäßen, beispielsweise Arteria mammaria interna, immer noch 92 %, während die bei Verwendung von venösen Gefäßen, wie beispielsweise der Vena saphena nur bei 60 % liegt. Über die frühen Verschlussraten innerhalb der ersten Tage und Wochen liegen bislang noch keine genauen Daten vor.

Im Stand der Technik sind bereits erste gentherapeutische Ansätze beschrieben, mit denen versucht wird, Bypass-Stenosen zu verhindern. So beschreiben beispielsweise Maeshima et al. (1998), Inhibition of Mesangial Cell Proliferation by E2F Decoy Oligodeoxynucleotide In Vitro and In Vivo, J. Clim. Invest., S. 2589-2597, ein synthetisiertes Oligonucleotid, das an den Transkriptionsfaktor E2F binden kann. Die Autoren konnten zeigen, dass nach Transfektion des Oligonucleotides in mesangiale Zellen die Expression des Transkriptionsfaktors E2F und des PCNA-Proteins (engl.: proliferating cell nuclear antigene) inhibiert und dadurch die Proliferation dieser Zellen verhindert wird. Die Firma Corgentech Inc., San Francisco, Vereinigte Staaten von Amerika, bieten ein solches Oligonucleotid unter der Bezeichnung "E2F Decoy" als Therapeutikum zur Prävention e i n e r B y p a s s-Stenose an; vgl. www1.corgentech.com/cgt/edifoligide.

Eine Inhibition der Funktion von E2F ist jedoch im Rahmen einer therapeutischen Anwendung äußerst kritisch. So ist bekannt, dass E2F nicht nur für eine pathologische Proliferation von Endothelzellen verantwortlich ist. Vielmehr handelt es sich bei E2F um einen zentralen Transkriptionsfaktor, der eine Vielzahl von Genen aktiviert, die für Zellzyklus regulierende Proteine kodieren, wie beispielsweise Dihydrofolatreduktase, c-myc, DNA-Polymerase α, cdc2, und PCNA. Durch das Einbringen eines die E2F-Funktion hemmenden Oligonucleotides wird deshalb auch verhindert, dass essentielle Reparaturmechanismen, z.B. in Zusammenhang mit der Wundheilung oder natürlichen Regeneration der Gefäße, stattfinden können. Patienten, die entsprechend transfizierte Bypass-Transplante erhalten, sind deshalb einem erhöhten Herzinfarktrisiko oder sonstigen Erkrankungen des Herzens ausgesetzt.

Die derzeit bekannten gentherapeutischen Maßnahmen im Zusammenhang mit kardiovaskulären Erkrankungen sind zusammenfassend dargestellt in Bonatti et al. (2002), Kardiovaskuläre Gentherapie - was kann der Chirurg derzeit davon erwarten?, J. Kardiol 9, Seiten 14 bis 20.

Große Herausforderungen an die moderne Medizin bzw. Arzneimittelforschung stellen ferner Erkrankungen der Atemwege, insbesondere der Lungen, dar. Für viele der hiervon betroffenen Patienten stellt eine Lungentransplantation die letzte therapeutische Möglichkeit dar, eine Verbesserung der Lebensqualität zu erreichen. Im Idealfall kann dadurch eine Steigerung der körperlichen Belastbarkeit, eine Entwöhnung von kontinuierlicher Sauerstofftherapie und damit eine deutliche Verbesserung der Lebensumstände erreicht werden. Weltweit gesehen nimmt die Zahl der durchgeführten Lungentransplantationen kontinuierlich zu.

Seit 1998 hat die Zahl der Patienten, die auf eine Lungentransplantation warten, um über 250 % zugenommen, die Zahl der durchgeführten Transplantationen nur um 70 % laut offizieller Statistik gemäß Euro Transplant, Leyden, Deutschland. D.h., es besteht ein zunehmender deutlicher Mangel an Spenderorganen. Dieser Mangel liegt teilweise daran, dass derzeit nur Organe aus Spendern verwendet werden, die zum Zeitpunkt der Organentnahme eine nahezu intakte Herz-Kreislauf-Situation haben, sog. "beating-heart-donors, BHD".

Eine Möglichkeit der Organknappheit zu entkommen eröffnet sich durch die Verwendung von Transplantationsorganen aus Spendern, deren Herz zum Entnahmezeitpunkt nicht mehr schlägt, sog. "non-heart-beating-donors, NHBD".

In beiden Fällen wird zwischen dem Zeitpunkt der Organentnahme und der Reimplantation im Empfänger das Organ nicht durchblutet, es ist ischämisch. Wie oben für die Bypass-Transplantate beschrieben, induziert diese Ischämie auch in dem Lungentransplantat eine starke Aktivierung des Endothels, so dass es zu einer Expression von inflammatorischen Adhäsionsmolekülen in der pulmonalen microvasculären Strombahn kommt. An diese inflammatorischen Adhäsionsmoleküle binden leucozytäre Zellen.

Im Weiteren kommt es zu einer transendothelialen Migration der leucozytären Zellen vom endovasculären Lumen ins Organparenchym mit einer folgenden Schädigung der pulmonalen Membranintegrität. In 20 bis 40 % der Fälle ist diese Schädigung so ausgeprägt, dass es zu einem klinisch relevanten Lungenödem kommt. Dies wird auch als primäres Transplantatversagen (engl.: "primary graft failure, PGF") bezeichnet. Das PGF ist mit einer deutlich erhöhten Co-Morbidität und damit einem verschlechterten primären Outcome der transplantierten Patienten verbunden; vgl. hierzu Meyers et al. (2005), Primary Graft Dysfunction and other Selected Complications of Lung Transplantation: A Single Center Experience of 983 Patients, J. Thorac. Cardiovasc. Surg. 129 (6), S. 1421-1429.

Aus tierexperimentellen Arbeiten ist bekannt, dass in Organen von NHBD die microvasculäre Endothelzellaktivierung noch fulminanter abläuft und damit das Outcome der transplantierten Patienten wesentlich schlechter ist, so dass diese Organe bislang nicht rekrutiert werden; vgl. Egan et al. (2004), Trigger for Intracellular Adhesion Molecule-1 Expression in Rat Lungs Transplanted from Non-Heart-Beating Donors, Ann. Thorac. Surg. 77 (3), S. 1048-1055. Könnte das Outcome von NHDBtransplantierten Organen dem Outcome von BHD-transplantierten Organen durch eine medikamentöse Therapiemöglichkeit angeglichen werden, so würde sich die Zahl der Patienten, die auf eine Lungentransplantation warten, deutlich verringern lassen, da mehr Organe für eine Transplantation zur Verfügung stünden.

Bislang gibt es keine spezifische Möglichkeit, die microvasculäre Endothelzellaktivierung in pulmonalen Transplantaten gezielt und spezifisch zu beeinflussen und somit die Inzidenz des PGF zu reduzieren. DeMeester et al. (1996), Attenuation of Rat Lung Isograft Reperfusion Injury with a Combination of Anti-ICAM1 and Anti-beta2 Integrin Monoclonal Antibodies, Transplantation 62 (10), S. 1477-1485, beschreiben die Blockierung von inflammatorischen Adhäsionsmolekülen im Tierexperiment mit Antikörpern. Obwohl mit dieser Vorgehensweise eine beachtliche Verbesserung des Transplantat-Outcomes erreicht wurde, konnte sich diese Therapie auf Grund erheblicher Antikörper-assoziierter Nebenwirkungen nicht durchsetzen.

Im Rahmen der Kardioplegie wird bei Operationen am offenen Herzen künstlich ein reversibler Herzstillstand induziert. Dieser Herzstillstand kann bspw. durch sog. kardioplegische Lösungen herbeigeführt werden, die eiskalt und pH neutral sind sowie Kalium sowie Magnesium enthalten. Während des Herzstillstandes kommt es zur Anreicherung von giftigen Stoffwechselprodukten, Kohlendioxid und Milchsäure im Herzen und dadurch zur Azidose. Diese Faktoren können in Zusammenwirkung mit der Ischämie die Herzmuskelzellen dauerhaft schädigen.

Poston et al. (1998) "Ex vivo gene therapy prevents chronic graft vascular disease in cardiac allografts", Journal of Thoracic and Cardiovascular Surgery, Seiten 386-396, Feeley (1999), "Pressure delivery of AS-ICAM-1 ODN with LFA-1 mAb reduces reperfusion injury in cardiac allografts", The Annals of Thoracic Sugery Seiten 119-124, Poston et al. (1999), "Antisense oligodeoxynucleotides prevent acute cardiac allograft rejection via a novel, nontoxic, highly efficient transfection method", Transplantation, Seiten 825-832, Feeley et al. (2000), "Optimization of ex vivo pressure mediated delivery of antisense oligodeoxynucleotides to ICAM-1 reduces reperfusion injury in rat cardiac allografts", Transplantation, Seiten 1067-1074 beschreiben die Verwendung von Antisense-Oligonukleotiden (AS-ODN) in der Kardioplegie.

Aufgabe der vorliegenden Erfindung ist es deshalb therapeutisch wirksame Substanzen bereitzustellen, mit denen die vorstehend beschriebenen Probleme im Zusammenhang mit der Kardioplegie vermieden werden können.

Insbesondere sollen solche Substanzen bereitgestellt werden, die sich einfach herstellen lassen und bspw. im Rahmen einer Herzoperation derart eingesetzt werden können, dass eine Stenose bzw. ein Transplantatversagen verhindert wird.

Diese Aufgabe wird erfindungsgemäß durch die Bereitstellung eines siRNA Moleküls zur Verwendung als therapeutisch wirksame Substanz in der Kardioplegie gelöst, das zumindest eine der Nucleotidsequenzen SEQ ID-Nr. 1-44 aus dem beiliegenden Sequenzprotokoll aufweist.

Den Erfindern ist es überraschenderweise gelungen über ein solches Nucleinsäuremolekül, das beispielsweise in biologische Zellen in Form eines sog. siRNA-Moleküls (small interfering RNA) transfiziert werden kann, die Ausbildung von Stenosen bzw. Schädigungen des Herzens im Rahmen der Kardioplegie zu verhindern. Derartige siRNA-Moleküle stellen doppelsträngige Strukturen aus Ribonucleinsäure dar, die einen posttranskriptionellen Prozess auslösen können, der als RNA-Interferenz (RNAi) bezeichnet wird und zur Stilllegung der Expression bestimmter Gene, dem sog. "gene silencing", führt. In eine biologische Zelle eingebracht werden die siRNA-Moleküle zu einem sog. Ribonucleasekomplex, dem sog. "RNA induced silencing complex" (RISC), rekrutiert. Dieser Komplex ist über das siRNA-Molekül in der Lage an im Wesentlichen komplementäre Strukturen, wie der mRNA eines transkribierten Genes zu binden und diese durch Endonucleaseaktivität zu degradieren. Dies führt im Ergebnis zur Inhibierung der Expression des entsprechenden Genes, das für die zum siRNA-Molekül komplementäre und degradierte mRNA kodiert.

Dabei war es bislang völlig unklar, dass über die Inhibition der Expression von Adhäsionsmolekülen die Ausbildung von Stenosen verhindert bzw. die pulmonale Membranintegrität erhalten werden kann. Insbesondere aber standen bislang keine Substanzen zur Verfügung, mit denen gezielt und ursächlich über diesen Mechanismus die Entstehung der koronaren Herzkrankheit oder ein primäres Transplantversagen bzw. eine Schädigung des Herzmuskels während der Kardioplegie verhindert werden konnte.

Die Erfinder haben erstmals ein Nucleinsäuremolekül entwickelt, mit dem dies möglich ist. Ein Nucleinsäuremolekül mit den Nucleotidsequenzen SEQ ID-Nr. 1 und Nr. 2, Nr. 3 und Nr. 4, Nr. 5 und Nr. 6, Nr. 7 und Nr. 8, Nr. 9 und Nr. 10, Nr. 11 und Nr. 12 oder Nr. 13 und Nr. 14 aus dem beiliegenden Sequenzprotokoll führt, in eine biologische Zelle eingebracht, überraschenderweise zur Inhibition der Expression des sog. intrazellulären Adhäsionsmoleküls 1 (ICAM-1; CD 54).

Ein Nucleinsäuremolekül, das die Nucleotidsequenzen SEQ ID-Nr. 15 und Nr. 16, Nr. 17 und Nr. 18, Nr. 19 und Nr. 20, Nr. 21 und Nr. 22, Nr. 29 und Nr. 30, Nr. 31 und Nr. 32, Nr. 33 und Nr. 34 oder Nr. 35 und Nr. 36 aus dem beiliegenden Sequenzprotokoll aufweist, führt nach dem Einbringen in eine biologische Zelle überraschenderweise zur Inhibition der Expression des sog. vasculären Zelladhäsionsmoleküls 1 (VCAM-1; CD 106).

Ein Nucleinsäuremolekül, das die Nucleotidsequenzen SEQ ID-Nr. 23 und Nr. 24, Nr. 25 und Nr. 26, Nr. 27 und Nr. 28, Nr. 37 und Nr. 38, Nr. 39 und Nr. 40, Nr. 41 und Nr. 42, Nr. 43 und Nr. 44 aus dem beiliegenden Sequenzprotokoll aufweist, führt, wenn es in eine biologische Zelle eingebracht wird, überraschenderweise zur Inhibition der Expression des endothelialen Adhäsionsproteins E-Selektin (CD-62E).

Die Expression dieser Gene ist bekanntermaßen dafür (mit-) verantwortlich, dass sich nach der Transplantation von venösen Gefäßen Graftstenosen ausbilden; vgl. Chello et al. (a.a.O.). Ferner ist bekannt, dass die Expression dieser Gene im Zusammenhang mit der Schädigung von Lungentransplantaten steht; vgl. DeMeester *et al.* (a.a.O.); Egan *et al.* (a.a.O.); Shreeniwas et al. (1996), Adhesion Molecules (E-Selectin and ICAM-1) in Pulmonary Allograft Rejection, Chest. 110 (5), S. 1143-1149. Im Stand der Technik ist es aber bisher nicht gelungen Maßnahmen oder Substanzen zu entwickeln, bei denen dieses Wissen therapeutisch genutzt wird.

Die Erfinder konnten hingegen zeigen, dass mittels eines siRNA-Moleküls, das bspw. zwei der vorstehend genannten Sequenzen aufweist, über die Inhibition der Expression von Adhäsionsmolekülen in den Gefäßen des zu transplantierenden bzw. zu behandelnden Organs die Offenheitsrate nach der Transplantation bzw. dem Abschluss einer kar-dioplegischen Herzoperation signifikant erhöht wird, bzw. die Membranintegrität weitgehend erhalten bleibt, wenn entsprechende Moleküle in die Zellen transfiziert wurden.

Derartige Moleküle können beispielsweise im Rahmen einer Bypassoperation verwendet werden. So können während der Operation explantierte Vena saphena-Abschnitte für ca. eine Stunde bis zur Verwendung als Bypass-Transplantat in beispielsweise einer Ringer-Lactat Lösung gelagert werden. Diese Phase kann für die Transfektion der Endothelzellen des Gefäßes mit den Nucleinsäuremolekülen als siRNA genutzt werden. Dabei werden in die Zellen ggf. siRNA-Moleküle verschiedenster Art in Form eines "Cocktails" hineintransfiziert. Die entsprechend behandelten Gefäßabschnitte können anschließend als Bypass transplantiert werden. Durch die Inhibition der Expression von Adhäsionsmolekülen in den biologischen Zellen, wird die Restenoserate im Bypass-Transplantat signifikant reduziert.

Die erfindungsgemäßen Moleküle können ferner im Rahmen einer Operation zur Lungentransplantation verwendet werden. Nach der Organentnahme wird die Lunge mit einer Konservierungslösung durchspült und bis zur Reimplantation im Empfänger für ca. sechs bis acht Stunden auf Eis gelagert. Diese Phase kann für die Transfektion der Endothelzellen der Lunge, d.h. der pulmonalen microvasculären Zellen mit den Nucleinsäuremolekülen als siRNA genutzt werden. Dazu können verschiedene siRNA-Moleküle, wie erwähnt, in Form eines "Cocktails" bereitgestellt und ggf. einer üblichen Konservierungslösung, z.B. Euro Collins, Perfadex, zugegeben werden. Dabei ist von Vorteil, dass die bekannten Transplantationsalgorithmen nicht verändert werden.

Die erfindungsgemäßen Moleküle können im Rahmen einer kardioplegischen Operation am offenen Herzen verwendet werden. Dazu werden die erfindungsgemäßen siRNA-Moleküle und ggf. in Form des "Cocktails" der kardioplegischen Lösung zugesetzt.

Die Erfinder haben damit das Prinzip des "gene silencings" auf die Kardioplegie übertragen. So beschreiben beispielsweise Nedbal et al. (2002), Antisense-mediated inhibition of ICAM-1 expression: a therapeutic strategy against inflammation of human periodontal tissue, Antisense & Nucleic Acid Drug Dev. 12(2), Seiten 71-78, die Verwendung von Antisense-Oligonucleotiden, die gegen ICAM-1 gerichtet sind, zur Behandlung von periodontalen Entzündungen.

Nishiwaki et al. (2003), Introduction of short interfering RNA to silence endogenous E-selectin in vascular endothelium leads to successful inhibition of leucocyte adhesion, Biochem. Biophys. Res. Commun. 310(4), Seiten 1062-1066, beschreiben siRNA-Moleküle, die gegen E-Selektin gerichtet sind und die Adhäsion von Leucozyten an das Endothelium verhindern.

Elbashir et al. (2001), Duplexes of 21-nucleotide RNAs mediate RNA interference in cultured mammalian cells, Nature 410 (6836), Seiten 494-498, beschreiben diverse siRNA-Moleküle, mit denen in verschiedenen Säugetierzelllinien endogene und heterologe Gene supprimiert werden können.

In der US 2004/0220129 A1 werden siRNA-Moleküle beschrieben, die gegen ICAM-1 gerichtet sind und für eine Verwendung zur Behandlung von entzündlichen und Autoimmunkrankheiten, Diabetes und Krebs vorgeschlagen werden.

In der WO 2004/045543 werden eine Vielzahl von siRNA-Molekülen ohne konkrete Angabe von Anwendungsfeldern oder klinischen Applikationen beschrieben.

In der WO 2004/065546 A2 werden siRNA-Moleküle beschrieben, die gegen ICAM-1 gerichtet sind und für eine Verwendung zur Behandlung von inflammatorischen und Autoimmunkrankheiten sowie Diabetes vorgeschlagen werden.

Die WO 03/099298 A2 beschreibt allgemein die siRNA-Technologie ohne jedoch konkrete Moleküle zu beschreiben.

Einen Überblick über die derzeitige Verwendung der siRNA-Technologie findet sich in Wadhwa et al. (2004), Know-how of RNA interference and its applications in research and therapy, Mutat. Res. 567(1), Seiten 71-84.

Die Erfindung umfasst nicht nur ein solches siRNA-Molekül, das aus den Sequenzen SEQ ID-Nr. 1 bis Nr. 44 besteht, sondern auch ein solches Nucleinsäuremolekül, das neben einer oder mehrerer der genannten Nucleotidsequenzen 5'- oder 3'-wärts weitere Nucleotide oder Verbindungen aufweist. Die Funktionalität des Nucleinsäuremoleküls als siRNA-Molekül wird dadurch nicht beeinträchtigt. Im Gegenteil kann durch das Anfügen zusätzlicher Nucleotide oder Verbindungen die Wirksamkeit des Moleküls erhöht werden, beispielsweise durch solche Nucleotidsequenzen, die eine Aufnahme des Nucleinsäuremoleküls in die zu behandelnden biologischen Zellen begünstigen oder Nucleaseresistenzen verleihen. Ferner können auch innerhalb der Nucleotidsequenzen SEQ ID-Nr. 1 bis SEQ ID-Nr. 44 vereinzelt Nucleotide ausgetauscht oder mutiert werden, ohne dass dadurch die Fähigkeit des Moleküls verloren geht, an eine im Wesentlichen komplementäre Nucleotidsequenz zu binden. So ist nämlich entscheidend, dass das erfindungsgemäße Nucleinsäuremolekül unter weitgehend stringenten Bedingungen an ein im Wesentlichen komplementäres Nucleinsäuremolekül, wie eine entsprechende mRNA, über ausreichend lange Abschnitte hinweg hybridisiert und es über die Aktivität des assemblierten RISC - im Falle der Verwendung eines siRNA-Moleküls - zur Spaltung der entsprechenden mRNA kommt. Unter stringenten Bedingungen werden in diesem Zusammenhang solche Bedingungen verstanden, bei denen ein unspezifisches Zusammenlagern von Nucleinsäuremolekülen verhindert wird. Beispielsweise werden solche stringenten Bedingungen durch Einstellen einer ausreichend hohen Salzkonzentration, eines bestimmten pH-Wertes, hohen Temperaturen, etc. geschaffen. Bei diesen Bedingungen hybridisieren solche Nucleinsäurestränge miteinander, die über eine ausreichende Länge hinweg Basenpaare miteinander ausbilden können.

Das erfindungsgemäße Nucleinsäuremolekül, das Bestandteil einer siRNA ist, kann dabei auch derart modifiziert sein, dass dadurch die Nucleaseresistenz innerhalb der Zelle erhöht ist. Dafür haben sich beispielsweise kurze Überhänge an beiden Strangenden bewährt, die aus DNA-Dimeren bestehen können. Besonders geeignet sind dabei Desoxythymidine (dt), die wiederholend vorzugsweise am 3'-Ende aber auch am 5'-Ende des erfindungsgemäßen Nucleinsäuremoleküls vorgesehen werden können.

Das erfindungsgemäße Nucleinsäuremolekül lässt sich einfach mittels im Stand der Technik bekannte Syntheseverfahren herstellen, vgl. hierzu Kretschmer-Kazemi Far R. und Sczakiel G. (2003), The activity of siRNA in mammalian cells is related to structural target accessibility: a comparison with antisense oligonucleotides, Nucleic Acids Res. 31(15), Seiten 4417 bis 4424.

Offenbart ist auch ein Nucleinsäuremolekül, das unter stringenten Bedingungen an das gleiche Nucleinsäuremolekül hybridisiert, an das das Nucleinsäuremolekül mit zumindest einer der Nucleotidsequenzen SEQ ID-Nr. 1 bis SEQ ID-Nr. 44 aus dem beiliegenden Sequenzprotokoll hybridisiert.

Die Erfinder haben ein therapeutisch wertvolles Nucleinsäuremolekül bzw. verschiedene Nucleotidsequenzen bereitgestellt, die es dem Fachmann ermöglichen, weitere Moleküle oder Sequenzen mit vergleichbaren Eigenschaften ohne großen Aufwand mittels Routinesyntheseverfahren herzustellen oder die angegebenen Moleküle oder Sequenzen ggf. durch Hinzufügung und Austausch einzelner Nucleotide zu modifizieren. Ein solches weiteres oder modifiziertes Nucleinsäuremolekül hybridisiert aber immer noch, ggf. mit geringfügigen zu tolerierenden Einbußen, an das gleiche Nucleinsäuremolekül, an das eines der Nucleotidsequenzen SEQ ID-Nr. 1 bis 44 hybridisiert, und löst damit gleichermaßen die erfindungsgemäße Aufgabe. Ein solches ggf. modifiziertes Nucleinsäuremolekül ist folglich ebenfalls Gegenstand der Erfindung.

Offenbart ist auch ein genetisches Konstrukt, das das oben beschriebene Nucleinsäuremolekül aufweist und zur Transfektion von biologischen Zellen, vorzugsweise Endothelzellen, einschließlich pulmonare microvasculäre Zellen, geeignet ist.

Ein solches genetisches Konstrukt zeichnet sich dadurch aus, dass es neben einer oder mehrerer der Nucleotidsequenzen SEQ ID-Nr. 1 bis SEQ ID-Nr. 44 oder hiervon abgeleiteten Sequenzen weitere Nucleotidsequenzen aufweist, die das Einbringen des Konstruktes in biologische Zellen, bspw. über Transfektion, begünstigen. Derartige weitere Nucleotidsequenzen oder Abschnitte, die der Transfektion dienen, sind dem Fachmann bekannt.

Beispiele hierfür sind Promotoren für die RNA-Polymerase, wie U6 oder H1; vgl. Brummelkamp et al. (2002), A system for stable expression of short interfering RNAs in mammalian cells. Science 296:550-553; oder Lee et al. (2002), Expression of small interfering RNAs targeted against HIV-1 rev transcripts in human cells. Nat Biotechnol 20:500-505, oder Miyagishi und Taira (2002), Development and application of siRNA expression vector, Nucleic Acids Res. [Suppl] 2002: 113-114.

Bei einem solchen Molekül ist die Eignung als Transfektionsmolekül nochmals optimiert. Das genetische Konstrukt eignet sich deshalb ganz besonders als therapeutisches Tool zur Inhibierung der Expression von Adhäsionsmolekülen.

Das genetische Konstrukt wird vorzugsweise so ausgestaltet, dass eine Transfektion von Endothelzellen einschließlich pulmonalen microvasculären Zellen begünstigt ist. So spielen nämlich diese Zellen eine Schlüsselrolle bei der Ausbildung von Stenosen bzw. der Schädigung der Gefäßmembranen bspw. bei Transplantaten. Eine verstärkte Expression von Adhäsionsmolekülen in diesen Zellen führt nämlich u.a. dazu, dass es zu einer transendothelialen Migration von Leucozyten kommt, wodurch die Integrität des Endothels beeinträchtigt wird und es zur Ausbildung von stenotischen Zuständen oder von PGF kommen kann. Hier schafft die vorliegende Erfindung wirksam Abhilfe. Wie die Erfinder nämlich feststellen konnten, wird durch die Inhibition der Expression der Adhäsionsmoleküle die transendotheliale Leukozytenmigration geblockt, so dass keine proinflammatorischen Stimuli für die Hyperplasie mehr gegeben sind.

Das genetische Konstrukt ist vorzugsweise ausgewählt aus der Gruppe bestehend aus RNA-Molekül, DNA-Molekül, Plasmid, Vektor, Virus. Diese Moleküle können einzel- oder doppelsträngig sein und ggf. Haarnadelstrukturen ausbilden. Unter ein genetisches Konstrukt fällt erfindungsgemäß auch ein vorstehend genanntes Molekül, das sich im Komplex mit Calciumphosphat befindet. Diese Maßnahme hat den Vorteil, dass über die Methode der sog. Calciumphosphatpräzipitation ein Einbringen des Nucleinsäuremoleküls bzw. genetischen Konstruktes in die biologischen Zellen besonders einfach möglich ist.

Gegenstand der vorliegenden Erfindung ist deshalb auch ein siRNA-Molekül, das ein Paar von RNA-Molekülen aufweist, die zumindest partiell gegeneinander hybridisiert sind, und jeweils Nucleotidsequenzen aufweisen, die ausgewählt sind aus der Gruppe bestehend aus: SEQ ID-Nr. 1 und 2; SEQ ID-Nr. 3 und 4; SEQ ID-Nr. 5 und 6; SEQ ID-Nr. 7 und 8; SEQ ID-Nr. 9 und 10; SEQ ID-Nr. 11 und 12; SEQ ID-Nr. 13 und 14; SEQ ID-Nr. 15 und 16; SEQ ID-Nr. 17 und 18; SEQ ID-Nr. 19 und 20; SEQ ID-Nr. 21 und 22; SEQ ID-Nr. 23 und 24; SEQ ID-Nr. 25 und 26; SEQ ID-Nr. 27 und 28; SEQ ID-Nr. 29 und 30; SEQ ID-Nr. 31 und 32; SEQ ID-Nr. 33 und 34; SEQ ID-Nr. 35 und 36; SEQ ID-Nr. 37 und 38; SEQ ID-Nr. 39 und 40; SEQ ID-Nr. 41 und 42; und SEQ ID-Nr. 43 und 44.

Wie die Erfinder herausgefunden haben, stellen diese siRNA-Moleküle besonders potente Tools zur Inhibition der Expression von Adhäsionsmolekülen dar und können deshalb zur Behandlung von Gefäßen, vorzugsweise venösen zu transplantierenden Gefäßen, eingesetzt werden.

Bevorzugt werden siRNA-Moleküle bereitgestellt, die gegenüber RNAsen stabilisiert sind. Diese Maßnahme hat den Vorteil, dass die Moleküle dadurch eine wesentlich höhere Lebensdauer aufweisen. So erhöht sich bspw. bei stabilisierten siRNA-Molekülen die Stabilität im Serum auf Stunden gegenüber von Minuten bei nicht stabilisierten siRNA-Molekülen. Zur Gewinnung von stabilisierten siRNA-Molekülen werden Letztere mittels dem Fachmann bekannten Verfahren chemisch modifiziert. Derartige Verfahren werden bspw. von der Firma Dharmacon, Inc., Chicago, Vereinigte Staaten von Amerika, unter der Bezeichnung siSTABLE^{™} v2, bzw. von der Firma Atugen, Berlin, Deutschland, unter der Bezeichnung AtuRNAi vertrieben. Stabilisierte siRNA-Moleküle werden auch beschrieben in Morrissey et al. (2005), Potent and persistent in vivo anti-HBV activity of chemically modified siRNAs, Nat. Biotechnol. 23(8), Seiten 1002 bis 1007; Chiu et al. (2003), siRNA Function in RNAi: A chemical modification analysis, RNA 9(9), Seiten 1034-1048.

Bei dem siRNA-Molekül handelt es sich vorzugsweise um ein hairpin-siRNA-Molekül. Solche hairpin-siRNA-Moleküle werden in die DNA der Empfängerzelle eingebaut und es kommt zu einer permanenten Expression der siRNA in der Zelle. Diese Maßnahme hat den Vorteil, dass die Expression der Adhäsionsmoleküle dauerhaft inhibiert wird, da die inhibierenden siRNA-Moleküle ständig "nachgeliefert" werden.

Offenbart ist ferner eine Zusammensetzung, die zumindest eines der vorstehend beschriebenen Nucleinsäuremoleküle, und/oder das vorstehend beschriebene genetische Konstrukt und/oder das vorstehend genannte siRNA-Molekül aufweist. Bei der Zusammensetzung handelt es sich vorzugsweise um eine physiologische Lösung zur Inkubation oder Konservierung von Organen, vorzugsweise Blutgefäßen oder Lungentransplantaten, die eine Puffersubstanz, Salze sowie ggf. weitere Hilfs-, Wirk-, Zusatz- oder Konservierungsstoffe aufweist.

In solch eine Zusammensetzung kann während oder nach der Operation das explantierte Organ, d.h. beispielsweise das zu transplantierende venöse Gefäß oder das Lungentransplantat, gegeben werden bei der Zusammensetzung kann es sich auch um eine kardioplegische Lösung handeln, die Kalium und Magnesium enthält. Mit bzw. in dieser Zusammensetzung kann dann die eigentliche Transfektion der darin enthaltenden Nucleinsäuremoleküle bzw. genetischen Konstrukte oder siRNA-Moleküle in die Zellen des

Gefäßes, Transplantates oder des offenen Herzens erfolgen. Wie oben erwähnt, kann die Zusammensetzung eine Mischung aus den erfindungsgemäßen Nucleinsäuremolekülen bzw. siRNA-Molekülen enthalten und so die Inhibition verschiedenster Adhäsionsmoleküle verhindern. Hilfs- oder Zusatzstoffe dienen der möglichst schonenden Lagerung bzw. Konservierung der Organe und sind dem Fachmann bekannt. Darunter fallen auch Stoffe, die die Transfektion vermitteln, wie bspw. Cellfectin, Lipofectamin, Optifect der Firma Invitrogen GmbH, Karlsruhe, Deutschland. Auch Konservierungsstoffe sind dem Fachmann bekannt, die ein Heranwachsen von kontaminierenden Mikroorganismen verhindern und beispielsweise Antibiotika umfassen können. Letztere können auch als Selektionsagenzien zur Überprüfung der erfolgreichen Transfektion dienen. Das Vorsehen von Wirkstoffen, wie beispielsweise weiteren therapeutisch aktiven Substanzen hat den Vorteil, dass das Organ parallel zur Transfektion auf andere bzw. zusätzliche Art und Weise therapeutisch vorbehandelt werden kann, was die dauerhafte Eignung des Transplantates als Bypass oder Atmungsorgan zusätzlich verbessern kann. Vorstehend genannte Stoffe und Salze sind einem Zellbiologen oder Transplantationsmediziner ebenfalls wohl bekannt.

Offenbart ist ferner eine Vorrichtung, insbesondere einen Stent, die bzw. der eine Beschichtung aufweist, wobei die Beschichtung das vorstehend beschriebene Nucleinsäuremolekül und/oder das genetische Konstrukt und/oder siRNA-Molekül und/oder die Zusammensetzung aufweist.

Stents werden meist in Form von kleinen Metallgitterröhrchen dazu verwendet, um verstopfte oder verengte Gefäße dauerhaft zu weiten und damit den Lumendurchmesser des Gefäßes zu vergrößern sowie ein Zusammenfallen des Gefäßes oder einen plötzlichen Wiederverschluss zu verhindern. Ein Stent ist also ein dehnbares, röhrenförmiges Geflecht, das als Gefäßstütze der verbesserten Offenhaltung von Blutgefäßen, beispielsweise im Herzen, dient.

Beschichtete Stents, die beispielsweise mit Hilfe eines Ballonkatheters an die entsprechende Stelle in dem zu behandelten Gefäß platziert werden, können derart präpariert werden, dass die sich in der Beschichtung befindlichen Nucleinsäuremoleküle bzw. siRNA-Moleküle etc. im Sinne eines "drug release" bzw. "drug eluting" an das umgebende Gefäß abgegeben werden und dort ihre therapeutische Wirkung entfalten. Mit derartigen Stents kann eine lokal begrenzte Applikation der Nucleinsäuremoleküle erfolgen. Die dafür eingesetzte Beschichtung kann aus liposomalen Nanopartikeln bestehen, in die die erfindungsgemäßen Nucleinsäuremoleküle mittels dem Fachmann bekannter physiko-chemischer Verfahren stabil integriert und ggf. mit einer zeitlichen Latenz an das zu behandelnde Gefäß abgegeben werden.

Es ist bevorzugt, wenn die Beschichtung der Vorrichtung bzw. des Stents durch biodegradierbare und/oder bioresorbierbare Polymere realisiert wird. Derartige Biomaterialien sind für chirurgische Anwendungen etabliert und dem Fachmann bekannt. Geeignete Biomaterialien umfassen Polysulfone (PSU), Polyethersulfone (PESU), Polyglycolide und Polylactide, wie bspw. Poly(dl-lactid-co-glycolid)[PLGA], Magnesium und Mischungen daraus. Bei diesen Materialien handelt es sich um besonders geeignete Beschichtungsmaterialien für Stents. Diese sorgen einerseits für den Erhalt der mechanischen Eigenschaften des Stents und für die Verbesserung von deren Biokompatibilität durch den Überzug. Andererseits kann das Polymer auch die erfindungsgemäßen Nucleinsäuremoleküle bzw. siRNA-Moleküle enthalten. Ein solches Polymer kann dann als Matrix für die gezielte Freisetzung der siRNA-Moleküle genutzt werden, die dann die Expression der entsprechenden Adhäsionsmoleküle inhibieren. Man kann in diesem Zusammenhang von sog. "Gene-Silencing-Stents" sprechen. Das biodegradierbare Polymer steht dabei mindestens so lange im Körper zur Verfügung, wie siRNA-Moleküle eluiert werden. Damit wird sichergestellt, dass das behandelte Gefäß nicht nur während der Operation in seiner Integrität unbeschädigt bleibt, sondern auch über einen bestimmten Zeitraum nach der Reimplantation im Empfänger.

Alternativ können die Stents auch vollkommen aus diesen Biomaterialien gebildet sein, die dann die erfindungsgemäßen Nucleinsäuremoleküle bzw. siRNA-Moleküle enthalten. Derartige resorbierbare Stents haben den Vorteil, dass sie die natürliche Gefäßarchitektur intakt lassen und auch z.B. dem Kardiochirurgen keine Ansatzstellen für Bypässe wegnehmen. Resorbierbare Stents ermöglichen u.U. auch das sog. "Plug-Sealing", d.h. das prophylaktische Stenting von hämodynamisch nicht relevanten Stenosen beim Nachweis instabiler Plugs. Die moderne Bildgebung kann derartige Strukturen zunehmend besser darstellen. Ein Überblick über bioresorbierbare Stents, insbesondere über Magnesiumstents, findet sich in Erne et al. (2005), The Road to Bioabsorbable Stents: Reaching Clinical Reality? Cardiovasc. Intervent. Radiol. 29.

Gegenstand der vorliegenden Erfindung ist vor diesem Hintergrund auch die Verwendung des Nucleinsäuremoleküls, das eine der Nucleotidsequenzen SEQ ID-Nr. 1 bis 44 aufweist bzw. eines Nucleinsäuremoleküls, das unter stringenten Bedingungen an das gleiche Nucleinsäuremolekül hybridisiert, als siRNA bzw. zur Herstellung letzterer.

Die Erfinder haben erstmals erkannt, dass ein Nucleinsäuremolekül mit den angegebenen Nucleotidsequenzen zum "gene silencing" verwendet werden kann und dadurch vorzugsweise die Inhibition der Expression von endothelialen Adhäsionsmolekülen bewirkt wird. Erfindungsgemäß wird ein Nucleinsäuremolekül, das die Nucleotidsequenzen SEQ ID-Nr. 1 und 2, Nr. 3 und 4, Nr. 5 und 6, Nr. 7 und 8, Nr. 9 und 10, Nr. 11 und 12 und Nr. 13 und 14 aus dem beiliegenden Sequenzprotokoll aufweist, oder ein Nucleinsäuremolekül mit vergleichbaren Hybridisierungseigenschaften, zur Inhibition der endothelialen Expression von ICAM-1 verwendet. Entsprechendes gilt für ein Nucleinsäuremolekül mit der Nucleotidsequenz SEQ ID-Nr. 15 und 16, Nr. 17 und 18, Nr. 19 und 20Nr. 21 und 22, Nr. 29 und 30, Nr. 31 und 32, Nr. 33 und 34; und Nr. 35 und 36 zur Inhibition der endothelialen Expression von VCAM-1, und für ein Nucleinsäuremolekül mit den Nucleotidsequenzen SEQ ID-Nr. 23 und 24, Nr. 25 und 26, Nr. 27 und 28, Nr. 37 und 38, Nr. 39 und 40, Nr. 41 und 42; und Nr. 43 und 44 zur Inhibition der endothelialen Expression von E-Selektin (C62-E).

Wie weiter oben erläutert, wird unter einem Nucleinsäuremolekül mit vergleichbaren Hybridisierungseigenschaften ein solches verstanden, das sich von den Nucleotidsequenzen SEQ ID-Nr. 1 bis 44 ableitet und gleichermaßen als siRNA-Molekül fungiert, wobei ggf. geringe Aktivitätseinbußen in Kauf genommen werden. So besteht die erfinderische Leistung nämlich nicht darin, eine spezifische Nucleotidsequenz anzugeben, die dann auf identische Art und Weise in ein entsprechendes Nucleinsäuremolekül umzusetzen ist, sondern vielmehr darin, dass Basissequenzen angegeben werden, die als Leitstrukturen für entsprechende Nucleinsäuremoleküle mit siRNA-Eigenschaften dienen. Die vorliegende Erfindung ist deshalb nicht auf Nucleinsäuremoleküle begrenzt, die ausschließlich aus einer der Nucleotidsequenzen SEQ ID-Nr. 1 bis 44 bestehen. Vielmehr sind derartige Nucleinsäuremoleküle umfasst, die von den angegebenen Sequenzen abgeleitete Sequenzen aufweisen und vergleichbare Hybridisierungseigenschaften unter stringenten Bedingungen haben.

Offenbart ist ferner ein Verfahren zur Inhibition der Expression von Adhäsionsmolekülen in biologischen Zellen, das folgende Schritte aufweist: (a) Bereitstellung der biologischen Zellen, vorzugsweise von Organen oder Organteilen, weiter vorzugsweise von Blutgefäßen, (b) Einbringen des oben beschriebenen Nucleinsäuremoleküls bzw. genetischen Konstruktes bzw. der siRNA-Moleküle in die biologischen Zellen, und fakultativ (c) Waschen und/oder Isolierung der biologischen Zellen.

Schritt (b) gelingt bspw. mittels üblicher Transfektionsmethoden, beispielsweise der Calciumphosphatpräzipitation, der Elektroporation, Mikroinjektion, Lipofektion, Polyfektion mittels Dendrimeren, rezeptorvermittelter Transfer etc.; vgl. hierzu beispielsweise Sambrook, J. und Russell D.W. (2001), Molecular Cloning - Laboratory Manual, Cold Spring Harbor Laboratory Press.

Mit diesem Verfahren lassen sich auf einfache Art und Weise sowohl isolierte biologische Zellen als auch Organe oder Organteile, wie Gefäßabschnitte oder Lungentransplantate *ex-vivo* derart behandeln, dass in den entsprechenden Zellen die Expression von Adhäsionsmolekülen, die im Zusammenhang mit dem Verschluss von Bypass-Transplantaten stehen, inhibiert wird. Mit diesem Verfahren wird deshalb dem Zellbiologen oder dem Physiologen sowie dem Chirurgen ein zielgerichtetes Verfahren an die Hand gegeben, mit dem sich eine gezielte (Vor-)Behandlung von biologischen Zellen bzw. Organteilen durchführen lässt. Die so behandelten Zellen bzw. Organteile können für wissenschaftliche Untersuchungen zellbiologischer Art aber auch zur anschließenden Transplantation, bspw. als Bypass oder Lunge, verwendet werden.

Bei dem Verfahren ist es bevorzugt, wenn das Einbringen in Schritt (b) mittels non-viraler transienter Transfektion erfolgt.

Diese Maßnahme hat den Vorteil, dass die infektiologische Komponente viraler Vektoren bei dieser Form der Transfektion entfällt. Die non-virale transiente Transfektion ist gleichermaßen äußerst effizient und mittels bekannter molekularbiologischer Maßnahmen auch während einer Herzoperation durchführbar.

Die Erfinder haben ebenfalls ein Gefäßtransplantationsverfahren entwickelt, das an einem Patienten durchgeführt werden kann und folgende Schritte aufweist: (a) Entnahme eines Gefäßes, vorzugsweise der Vena saphena magna, bei dem Patienten, (b) Einbringen des wie oben beschriebenen Nucleinsäuremoleküls oder des genetischen Konstruktes oder der siRNA-Moleküle in die Zellen des Gefäßes, vorzugsweise mittels non-viraler transienter Transfektion, und (c) Transplantation bzw. Implantation des behandelten Gefäßes, vorzugsweise zur Herstellung eines Bypasses.

Im Gegensatz zu den bisher durchgeführten Gefäßtransplantationsverfahren ist hier von Vorteil, dass durch die Behandlung des Gefäßes in den dortigen Endothelzellen durch das Einbringen der erfindungsgemäßen Nucleinsäuremoleküle oder genetischen Konstrukte bzw. siRNA-Moleküle die Expression von Adhäsionsmolekülen inhibiert wird. Das anschließend transplantierte Gefäß zeigt im Vergleich zu unbehandelten Transplantaten eine wesentlich geringere Restenoserate. Im Rahmen der Inkubation gemäß Schritt (b) erfolgt eine Transfektion der Endothelzellen beispielsweise über nicht-virale liposomale Transfektion. Diese führt zu einer transienten Protektion des Bypass-Transplantates, die insbesondere während der ersten kritischen Stunden der Reperfusionsphase einen Schutz des Bypassendothels bewirkt und somit zu einer Verringerung der frühen Restenoserate beiträgt.

Vor diesem Hintergrund erfolgt Schritt (c) nach Abschluss von Schritt (a) in einem zeitlichen Abstand von bis zu fünf Stunden, vorzugsweise von maximal einer Stunde.

Diese Maßnahme hat den Vorteil, dass sichergestellt wird, dass das entnommene Gefäß keine Vitalitätseinbußen erleidet, sondern vielmehr dessen physiologische Funktionen voll erhalten bleiben. Minimale Vitalitätseinbußen sind jedoch zu tolerieren.

Bislang gibt es keine klinisch praktikablen Therapiemöglichkeiten zur Blockierung der endothelialen Adhäsionsmolekülexpression und damit zur Protektion der Gefäßtransplantate. Im Stand der Technik werden vereinzelt experimentelle Ansätze beschrieben, bei denen eine Protektion von Gefäßtransplantaten über die Applikation von Antikörpern gegen Adhäsionsmoleküle oder über eine hypertherm induzierte Freisetzung von protektiv wirksamen Hitzeschockproteinen anvisiert wird. Derartige Ansätze haben sich allerdings nicht bzw. als pharmakologisch äußerst problematisch umsetzbar erwiesen.

Die Erfinder stellen deshalb ein wichtiges Tool und Verfahren bereit, das zur Behandlung der koronaren Herzkrankheit eingesetzt werden kann und insbesondere optimal in das chirurgische OP-Management bei Bypassoperationen integriert werden kann.

Die Erfinder haben ferner ein Lungentransplantationsverfahren entwickelt, das an einem Patienten durchgeführt werden kann und folgende Schritte aufweist: (a) Entnahme der Lunge aus einem Spender, (b) Einbringen des wie oben beschriebenen Nucleinsäuremoleküls oder des genetischen Konstruktes oder des siRNA-Moleküls in die pulmonalen microvasculären Zellen, vorzugsweise mittels non-viraler transienter Transfektion, und (c) Transplantation bzw. Implantation der Lunge in den Patienten. Bei dem Spender kann es sich sowohl um einen "Beating-Heart-Donor" (BHD) oder um einen "Non-Heart-Beating-Donor" (NHBD) handeln.

Schritt (c) erfolgt vorzugsweise nach Abschluss von Schritt (a) in einem zeitlichen Abstand von ca. sechs bis acht Stunden. Diese Maßnahme hat den Vorteil, dass der Zeitraum der Lagerung der Lunge in einer Konservierungslösung optimal für die Transfektion der siRNA-Moleküle genutzt wird.

Offenbart ist außerdem ein Lungentransplantatbehandlungs- bzw. -konservierungsverfahren, das folgende Schritte aufweist: (a) Bereitstellung eines Lungentransplantates, und (b) Einbringen des wie oben beschriebenen Nucleinsäuremoleküls oder des genetischen Konstruktes oder des siRNA-Moleküls in die pulmonalen microvasculären Zellen, vorzugsweise mittels non-viraler transienter Transfektion.

Offenbart ist schließlich ein Verfahren zur Behandlung des offenen Herzens im Rahmen der Kardioplegie, das folgende Schritte aufweist: (a) Bereitstellung eines Patienten mit offenem Herzen, (b) Einbringen des wie oben beschriebenen Nucleinsäuremoleküls oder des genetischen Konstruktes oder des siRNA-Moleküls in die Endothelzellen des Herzens, vorzugsweise mittels non-viraler transienter Transfektion.

Die vorliegende Erfindung wird nun anhand von Ausführungsbeispielen erläutert, die rein illustrativ sind und die Reichweite der Erfindung keinesfalls einschränken. Dabei wird auf die beigefügten Figuren Bezug genommen, in denen Folgendes zu sehen ist:
- Fig. 1: zeigt das Ergebnis einer durchflusszytometrischen Analyse, mit der die Inhibition der ICAM-Expression in Endothelzellen mittels erfindungsgemäßer Nucleinsäuremoleküle erreicht wurde.
- Fig. 2: zeigt das Ergebnis eines vergleichbaren Experimentes, in dem in Endothelzellen die Inhibition der VCAM-1-Expression über erfindungsgemäße Nucleinsäuremoleküle erreicht wurde.
- Fig. 3: zeigt das Ergebnis eines weiteren vergleichbaren Experimentes, in dem in Endothelzellen die Inhibition der Expression von E-Selektin (CD-62E) mittels erfindungsgemäßer Nucleinsäuremoleküle erreicht wurde.
- Fig. 4: zeigt das Ergebnis einer durchflusszytometrischen Analyse, mit der die Inhibition der ICAM-Expression in pulmonalen microvasculären Zellen aus humanen Lungen mittels erfindungsgemäßer Nucleinsäuremoleküle erreicht wurde.
- Fig. 5: zeigt das Ergebnis eines vergleichbaren Experimentes, in dem in pulmonalen microvasculären Zellen die Inhibition der VCAM-1-Expression über erfindungsgemäße Nucleinsäuremoleküle erreicht wurde.
- Fig. 6: zeigt das Ergebnis eines vergleichbaren Experimentes, in dem in pulmonalen microvasculären Zellen die Inhibition der Expression von E-Selektin über erfindungsgemäße Nucleinsäuremoleküle erreicht wurde.

### Ausführungsbeispiele

### Nucleinsäuremoleküle

Die im Rahmen der Experimente, die zu der Erfindung geführt haben, verwendeten Nucleinsäuremoleküle wurden als Oligonucleotide nach dem Fachmann gängigen Verfahren synthetisiert. Zur Formierung eines doppelsträngigen siRNA-Moleküls wurde ein Sinn-Strang mit dem jeweiligen Gegensinn-Strang unter im Stand der Technik bekannten Bedingungen hybridisiert.

Die Synthese der Oligonucleotide sowie die Formierung von siRNA ist beispielsweise beschrieben in Kretschmer-Kazemi (a.a.O.), dort insbesondere im Material- und Methodenteil.

Die nachstehende Tabelle gibt einen Überblick über die verwendeten erfindungsgemäßen Nucleinsäuremoleküle:

**Tabelle 1: Erfindungsgemäße Nucleinsäuremoleküle**

| siRNA | Target | Oligonucleotide | SEQ ID-Nr. |
|---|---|---|---|
| 1 | ICAM-1 | 5'-GCCUCAGCACGUACCUCUA-3' (Sinn) | 1 |
| | | 5'-UAGAGGUACGUGCUGAGGC-5' (Gegensinn) | 2 |
| 2 | ICAM-1 | 5'-CCCUUGAUGAUAUGUAUUU-3' (Sinn) | 3 |
| | | 5'-AAAUACAUAUCAUCAAGGG-5' (Gegensinn) | 4 |
| 3 | ICAM-1 | 5'-GCCAGCUUAUACACAAGAA-3' (Sinn) | 5 |
| | | 5'-UUCUUGUGUAUAAGCUGGC-5' (Gegensinn) | 6 |
| 4 | ICAM-1 | 5'-GAUCAAGAAAUACAGACUA-3' (Sinn) | 7 |
| | | 5'-UAGUCUGUAUUUCUUGAUC-5' (Gegensinn) | 8 |
| 5 | ICAM-1 | 5'-CAAGAAAUACAGACUACAA-3' (Sinn) | 9 |
| | | 5'-UUGUAGUCUGUAUUUCUUG-5' (Gegensinn) | 10 |
| 6 | ICAM-1 | 5'-AGACUACAACAGGCCCAAA-3' (Sinn) | 11 |
| | | 5'-UUUGGGCCUGUUGUAGUCU-5' (Gegensinn) | 12 |
| 7 | ICAM-1 | 5'-GUCAGAUACAACAGCAUUU-3' (Sinn) | 13 |
| | | 5'-AAAUGCUGUUGUAUCUGAC-5' (Gegensinn) | 14 |
| 8 | VCAM-1 | 5'-GAUAGAUAGUCCACUGAAU-3' (Sinn) | 15 |
| | | 5'-AUUCAGUGGACUAUCUAUC-5' (Gegensinn) | 16 |
| 9 | VCAM-1 | 5'-GGAUACGGAUAUGAAAUCU-3' (Sinn) | 17 |
| | | 5'-AGAUUUCAUAUCCGUAUCC-5' (Gegensinn) | 18 |
| 10 | VCAM-1 | 5'-GUACGCAAACACUUUAUGU-3' (Sinn) | 19 |
| | | 5'-ACAUAAAGUGUUUGCGUAC-5' (Gegensinn) | 20 |
| 11 | VCAM-1 | 5'-AAUGCAACUCUCACCUUAA-3' (Sinn) | 21 |
| | | 5'-UUAAGGUGAGAGUUGCAUU-5' (Gegensinn) | 22 |
| 12 | E-Selektin | 5'-GACCAUCAAUAAUUACACU-3' (Sinn) | 23 |
| | | 5'-AGUGUAAUUAUUGAUGGUC-5' (Gegensinn) | 24 |
| 13 | E-Selektin | 5'-ACGUGUAAAGCUGUGACAU-3' (Sinn) | 25 |
| | | 5'-AUGUCACAGCUUUACACGU-5' (Gegensinn) | 26 |
| 14 | E-Selektin | 5'-UUAAAGAGAGUGGAGCCUGGU-3' (Sinn) | 27 |
| | | 5'-ACCAGGCUCCACUCUCUUUAA-5' (Gegensinn) | 28 |
| 15 | VCAM-1 | 5'-GGAGGAUACGGAUAUGAAA-3' (Sinn) | 29 |
| | | 5'-UUUCAUAUCCGUAUCCUCC-3' (Gegensinn) | 30 |
| 16 | VCAM-1 | 5'-GAGCUAAAUUACACAUUGA-3' (Sinn) | 31 |
| | | 5'-UCAAUGUGUAAUUUAGCUC-3' (Gegensinn) | 32 |
| 17 | VCAM-1 | 5'-CAUCUACGCUGACAAUGAA-3' (Sinn) | 33 |
| | | 5'-UUCAUUGUCAGCGUAGAUG-3' (Gegensinn) | 34 |
| 18 | VCAM-1 | 5'-CUCUAUAUUUAGAUUGUUA-3' (Sinn) | 35 |
| | | 5'-UAACAAUCUAAAUAUAGAG-3' (Gegensinn) | 36 |
| 19 | E-Selektin | 5'-GGUUGAAUGCACCACUCAA-3' (Sinn) | 37 |
| | | 5'-UUGAGUGGUGCAUUCAACC-3' (Gegensinn) | 38 |
| 20 | E-Selektin | 5'-UGGUAGAAUUGGAGAGUAA-3' (Sinn) | 39 |
| | | 5'-UUACUCUCCAAUUCUACCA-3' (Gegensinn) | 40 |
| 21 | E-Selektin | 5'-CAGUGUGGUUUGUGUUUGA-3' (Sinn) | 41 |
| | | 5'-UCAAACACAAACCACACUG-3' (Gegensinn) | 42 |
| 22 | E-Selektin | 5'-CGGAAGCUAUGACUUAUGA-3' (Sinn) | 43 |
| | | 5'-UCAUAAGUCAUAGCUUCCG-3' (Gegensinn) | 44 |
| 23 | Negativkontrolle | 5'-UUCUCCGAACGUGUCACGU-3' (Sinn) | 45 |
| | | 5'-ACGUGACACGUUCGGAGAA-5' (Gegensinn) | 46 |

### Patienten

Gefäßproben, d.h. Sektionen der Vena saphena magna, wurden aus Patienten gewonnen, die einer aortokoronaren Bypassoperation (engl.: coronary artery bypass grafting, CABG) unterzogen wurden. Derartige Sektionen werden nach einer solchen Operation üblicherweise verworfen. Die Patienten gaben vorab ihr Einverständnis zur wissenschaftlichen Verwendung des Gefäßabschnittes.

Die Durchführung der Experimente wurde zuvor von der Ethikkommission der Universität Tübingen genehmigt.

### Isolierung und Kultivierung der Endothelzellen aus der Vena saphena magna

Die Gewinnung und Kultivierung der Zellen erfolgte gemäß bereits publizierter Arbeitsprotokolle; vgl. Nachman, R.L. und Jaffe, E.A. (2004), Endothelial cell culture: beginnings of modern vascular biology, J. Clin. Invest. 114(8), Seiten 1037 - 1040, und Jaffe et al. (1973), Culture of human endothelial cells derived from umbilical veins. Identification by morphologic and immunologic criteria, J. Clin. Invest. 52(11), Seiten 2745 - 2756.

Kurz, sämtliche Kulturplatten und -flaschen (Becton Dickinson GmbH, Heidelberg, Deutschland) wurden über Nacht mit 40 % Kollagen (Collagen G, Biochrom, Indiana, USA) beschichtet. Die gesamte Vene wurde zuerst in RPMI 1640 Puffer (Cambrex Bio Science Verviers, S.p.r.l., Verviers, Belgien) und 0,5 %/ml Gentamycin (Invitrogen GmbH, Karlsruhe, Deutschland) inkubiert. Anschließend wurde die Vene mit Puffer (137 mM in NaCl, 5,4 mM KCl, 4,2 mM NaHCO₃, 5 mM D-Glucose in 500 ml H₂O, pH 7,3, steril), gespült. Die Gewinnung der Endothelzellen erfolgte durch Verdau mit 0,1 % Kollagenase (PAA Laboratories GmbH, Cölbe, Deutschland) in phosphatgepufferter Saline (PBS) und weiterer Kultivierung in EGM-2 (+bullet kit, Cambrex Bio Science Verviers). Die Zellen wurden nach Erreichen der Konfluenz durch Behandlung mit Trypsin (Detach Kit, PromoCell GmbH, Heidelberg, Deutschland) voneinander getrennt. Für sämtliche Experimente wurden Zellen aus der dritten Passage verwendet. Um zu überprüfen, dass keine Kontamination mit Mykoplasmen erfolgte, wurden Tests mit der DAPI-Methode (SERVA Elektrophoresis GmbH, Heidelberg, Deutschland) durchgeführt. Die Reinheit der isolierten venösen Endothelzellen wurde durch Färbung gegen den Willebrand-Faktor, VEGFR-2, Tie-2 überprüft.

### Pulmonale microvasculäre Zellen

Die humanen pulmonalen microvasculären Zellen wurden bezogen von Cambrex Bio Science Wokingham, Ltd., Berkshire, England.

### Transfektion der isolierten Endothelzellen

Sämtliche Transfektionen wurden unter RNAse-freien Bedingungen durchgeführt. Die isolierten Endothelzellen wurden in kollagenisierten 12-well-Platten (Greiner Bio-One GmbH, Frickenhausen, Deutschland) ohne Antibiotika kultiviert. Nach Erreichen der Konfluenz (70-80 %) wurden die isolierten Endothelzellen mit hybridisierter siRNA unter Verwendung von 2,01 µl/ml Cellfectin (Invitrogen GmbH, Karlsruhe, Deutschland) transfiziert. Vier verschiedene Zellbehandlungen, jeweils in Doppelansätzen, wurden parallel getestet: 1. nicht-stimulierte isolierte Endothelzellen/pulmonale microvasculäre Zellen, nicht-transfiziert; 2. stimulierte (TNF-α, 2,5 ng/ml bzw. 5 ng/ml) isolierte Endothelzellen/pulmonale microvasculäre Zellen, nicht transfiziert; 3. stimulierte mit erfindungsgemäßen siRNA-Molekülen transfizierte isolierte Endothelzellen/pulmonale microvasculäre Zellen; 4. stimulierte mit unspezifischer randomisierter siRNA (QIAGEN GmbH, Hilden, Deutschland) transfizierte isolierte Endothelzellen/pulmonale microvasculäre Zellen.

Die Transfektionszeit betrug 2 Stunden und die Experimente wurden viermal wiederholt. Nach 9 Stunden wurden sämtliche isolierten Endothelzellen/pulmonale microvasculäre Zellen mit TNF-α (2,5 ng/ml bzw. 5 ng/ml) (Immunotools, Friesoythe, Deutschland) für 15 Stunden stimuliert.

### Durchflusszytometrische Analyse (engl. Fluorescence Activating Cell Sorting) (FACS)

Nach TNF-α Stimulierung wurden sämtliche Ansätze von isolierten und ggf. transfizierten Endothelzellen/pulmonale microvasculäre Zellen mit EGM-2-Medium gewaschen. Das unspezifische Binden von Antikörpern wurde durch FCS (5 %, pH 7,4) blockiert. Zur Färbung der ggf. transfizierten isolierten Endothelzellen/pulmonale microvasculäre Zellen wurde ein PE-markierter Antikörper gegen die jeweiligen Adhäsionsmoleküle (Becton Dickinson GmbH, Heidelberg, Deutschland), d.h. gegen ICAM-1, VCAM-1 bzw. E-Selektin (CD-62E) verwendet (4°C, 1 Stunde). Nach dem Waschen und Ablösen wurden die Zellen mit 2,5 % Paraformaldehyd in PBS fixiert. Die durchflusszytometrischen Analysen (5000 Zellen/ Messung) wurden in einem FACScan^{™} (Becton Dickinson GmbH, Heidelberg, Deutschland) durchgeführt und mit der CellQuestPro-Software (Becton Dickinson GmbH, Heidelberg, Deutschland) kalkuliert).

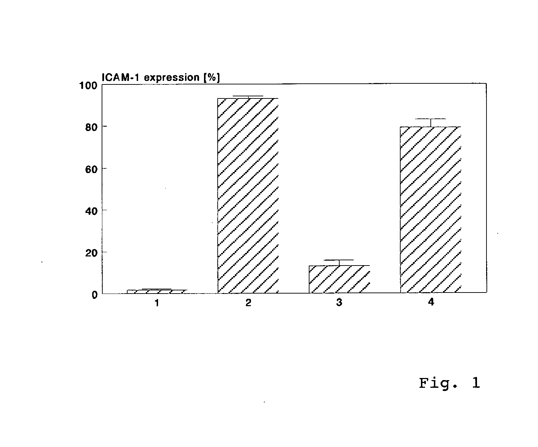

Das Ergebnis solcher Experimente ist in den Figuren Fig. 1 bis 6 dargestellt.

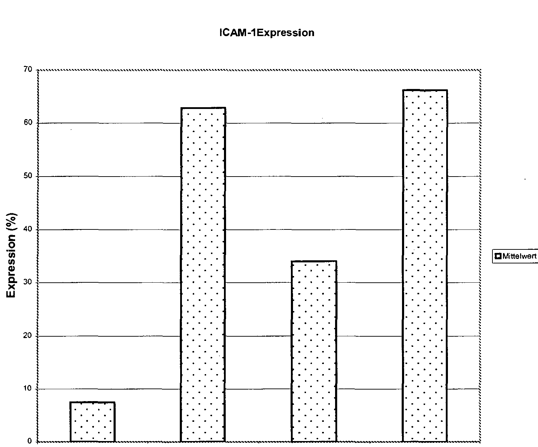

In den Figuren 1 und 4 sind repräsentative Ergebnisse der Experimente dargestellt, bei denen die isolierten Endothelzellen (Fig. 1) bzw. pulmonale microvasculäre Zellen (Fig. 4) mit einer der erfindungsgemäßen siRNA transfiziert wurden, die gegen ICAM-1 gerichtet war, d.h. mit einem siRNA-Molekül mit der Nr. 1 bis 7 gemäß der Tabelle 1. Die Färbung der Zellen in der FACS-Analyse erfolgte entsprechend mit einem Antikörper, der gegen humanes ICAM-1 gerichtet war. Das Ergebnis sah für jedes der siRNA-Moleküle vergleichbar aus.

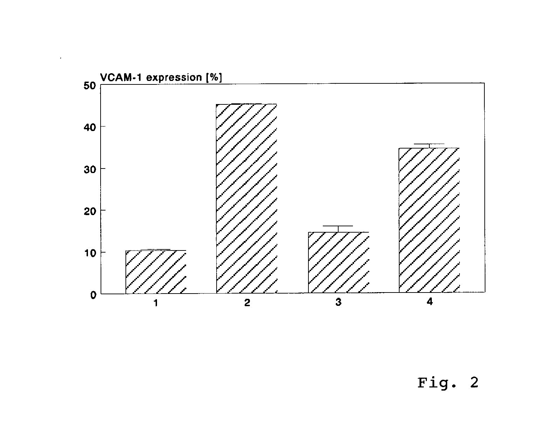

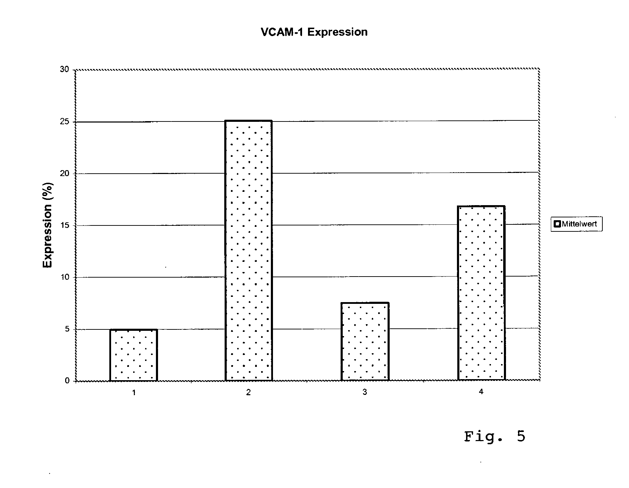

In den Figuren 2 und 5 sind repräsentative Ergebnisse der Experimente dargestellt, bei denen die isolierten Endothelzellen (Fig. 2) bzw. pulmonale microvasculäre Zellen (Fig. 5) mit einer der erfindungsgemäßen siRNA transfiziert wurden, die gegen VCAM-1 gerichtet war, d.h. mit einem siRNA-Molekül mit der Nr. 8 bis 11 bzw. 15 bis 18 gemäß der Tabelle 1. Die Färbung der Zellen in der FACS-Analyse erfolgte entsprechend mit einem Antikörper, der gegen humanes VCAM-1 gerichtet war. Auch hier sah das Ergebnis für jedes der siRNA-Moleküle vergleichbar aus.

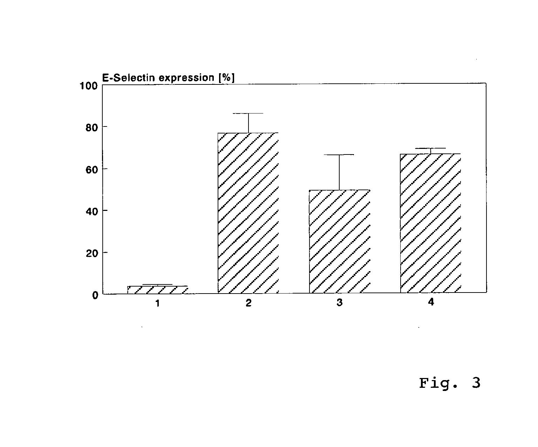

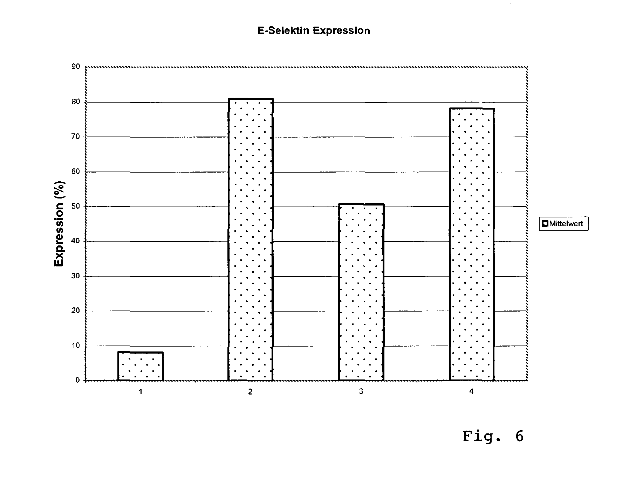

In den Figuren 3 und 6 sind repräsentative Ergebnisse der Experimente dargestellt, bei denen die isolierten Endothelzellen (Fig. 3) bzw. pulmonale microvasculäre Zellen (Fig. 6) mit einer der erfindungsgemäßen siRNA transfiziert wurden, die gegen E-Selektin gerichtet war, d.h. mit einem siRNA-Molekül mit der Nr. 12 bis 14 bzw. 19 bis 22 gemäß der Tabelle 1. Die Färbung der Zellen in der FACS-Analyse erfolgte entsprechend mit einem Antikörper, der gegen humanes E-Selektin gerichtet war. Das Ergebnis sah für jedes der siRNA-Moleküle vergleichbar aus.

In den Ansätzen (1) ist jeweils die Negativkontrolle, d.h. ohne Transfektion und ohne Stimulation, dargestellt. In den Ansätzen (2) sind jeweils die Positivkontrollen, d.h. ohne Transfektion aber mit Stimulation durch TNF-α, dargestellt. In den Ansätzen (3) sind die Transfektionen mit der spezifischen siRNA in TNF-α stimulierte Endothelzellen/pulmonale microvasculäre Zellen dargestellt. In den Ansätzen (4) sind Kontrollen dargestellt, bei denen die Endothelzellen/pulmonalen microvasculären Zellen mit TNF-α stimuliert und mit der unspezifischen siRNA (Nr. 23 gemäß Tabelle 1; SEQ ID-Nr. 45 und Nr. 46) transfiziert wurden.

In sämtlichen Ansätzen (3) ist zu sehen, dass die Transfektion der Zellen mit erfindungsgemäßer spezifischer siRNA in den entsprechenden Endothelzellen/pulmonalen microvasculären Zellen zu einer drastischen Inhibierung der Expression der jeweiligen Adhäsionsmoleküle geführt hat; vgl. mit Ansätzen (2).

Diese Inhibierung in den Endothelzellen ist im Falle von ICAM-1 ganz besonders deutlich ausgefallen. Das Expressionsniveau wurde hier durch die Behandlung mit der erfindungsgemäßen siRNA um nahezu 90 % inhibiert. Im Falle von VCAM-1 wurde das Expressionsniveau um nahezu 70 % reduziert. Im Falle von E-Selektin (CD-62E) wurde das Expressionsniveau um über 40 % reduziert.

I den pulmonalen microvasculären Zellen wurde das Expressionsniveau von ICAM-1 durch die Behandlung mit der erfindungsgemäßen siRNA um knapp 50 % inhibiert. Im Falle von VCAM wurde das Expressionsniveau um über 70 % reduziert. Im Falle von E-Selektin wurde eine Reduktion um mehr als 30 % beobachtet.

Die Erfinder konnten demnach demonstrieren, dass die erfindungsgemäßen Nucleinsäuremoleküle äußerst potente Tools für die Inhibition der Expression von Adhäsionsmolekülen in biologischen Zellen darstellen und deshalb zur Behandlung von Transplantaten besonders geeignet sind.

## Patentansprüche

1. siRNA-Molekül, das zumindest eine der Nucleotidsequenzen SEQ ID-Nr. 1 bis Nr. 44 aus dem beiliegenden Sequenzprotokoll aufweist, zur Verwendung als therapeutisch wirksame Substanz in der Kardioplegie.

2. siRNA-Molekül zur Verwendung nach Anspruch 1, das ein Paar von RNA-Molekülen aufweist, die zumindest partiell gegeneinander hybridisiert sind und jeweils Nucleotidsequenzen aufweisen, die ausgewählt sind aus der Gruppe bestehend aus: SEQ ID-Nr. 1 und 2; SEQ ID-Nr. 3 und 4; SEQ ID-Nr. 5 und 6; SEQ ID-Nr. 7 und 8; SEQ ID-Nr. 9 und 10; SEQ ID-Nr. 11 und 12; und SEQ ID-Nr. 13 und 14, SEQ ID-Nr. 15 und 16; SEQ ID-Nr. 17 und 18; SEQ ID-Nr. 19 und 20; SEQ ID-Nr. 21 und 22; SEQ ID-Nr. 29 und 30; SEQ ID-Nr 31 und 32; SEQ ID-Nr. 33 und 34; und SEQ ID-Nr. 35 und 36, SEQ ID-Nr. 23 und 24; SEQ ID-Nr. 25 und 26; SEQ ID-Nr. 27 und 28; SEQ ID-Nr. 37 und 38; SEQ ID-Nr. 39 und 40; SEQ ID-Nr. 41 und 42; und SEQ ID-Nr. 43 und 44.

## Claims

1. siRNA molecule comprising at least one of the nucleotide sequences of SEQ ID No. 1 to No. 44 from the accompanying sequence listing for a use as therapeutically effective substance in the cardioplegia.

2. siRNA molecule for a use according to claim 1, comprising a pair of RNA molecules which are at least in parts hybridizable against each other, and each comprising nucleotide sequences which are selected from a group consisting of SEQ ID No. 1 and 2; SEQ ID No. 3 and 4; SEQ ID No. 5 and 6; SEQ ID No. 7 and 8; SEQ ID No. 9 and 10; SEQ ID No. 11 and 12; and SEQ ID No. 13 and 14; SEQ ID No. 15 and 16; SEQ ID No. 17 and 18; SEQ ID No. 19 and 20; SEQ ID No. 21 and 22; SEQ ID No. 29 and 30; SEQ ID No. 31 and 32; SEQ ID No. 33 and 34; and SEQ ID No. 35 and 36, SEQ ID No. 23 and 24; SEQ ID No. 25 and 26; SEQ ID No. 27 and 28; SEQ ID No. 37 and 38; SEQ ID No. 39 and 40; SEQ ID No. 41 and 42; and SEQ ID No. 43 and 44.

## Revendications

1. Molécule d'ARNsi qui présente au moins l'une des séquences nucléotidiques SEQ ID N° : 1 à 44 du protocole de séquences ci-joint, pour son utilisation en tant que substance thérapeutiquement efficace dans la cardioplégie.

2. Molécule d'ARNsi pour son utilisation selon la revendication 1, qui présente une paire de molécules d'ARN qui sont hydridées au moins partiellement les unes par rapport aux autres et présentent respectivement des séquences nucléotidiques qui sont choisies dans le groupe comprenant : SEQ ID N° : 1 et 2 ; SEQ ID N° : 3 et 4 ; SEQ ID N° : 5 et 6 ; SEQ ID N° : 7 et 8 ; SEQ ID N° : 9 et 10 ; SEQ ID N° : 11 et 12 ; et SEQ ID N° : 13 et 14 ; SEQ ID N° : 15 et 16 ; SEQ ID N° : 17 et 18 ; SEQ ID N° : 19 et 20 ; SEQ ID N° : 21 et 22 ; SEQ ID N° : 29 et 30 ; SEQ ID N° : 31 et 32 ; SEQ ID N° : 33 et 34 ; SEQ ID N° : 35 et 36 ; SEQ ID N° : 23 et 24 ; SEQ ID N° : 25 et 26 ; SEQ ID N° : 27 et 28 ; SEQ ID N° : 37 et 38 ; SEQ ID N° : 39 et 40 ; SEQ ID N° : 41 et 42 ; et SEQ ID N° : 43 et 44.


### SEQUENZPROTOKOLL

<110> Eberhard-Karls-Universität Tübingen
<120> siRNA-Moleküle zur Behandlung von Blutgefäßen
<130> 5402P338WO
<140>
   <141>
<160> 46
<170> PatentIn Ver. 2.1
<210> 1
   <211> 19
   <212> RNA
   <213> Künstliche Sequenz
<220>
   <223> Beschreibung der künstlichen Sequenz: Oligonucleotid
<400> 1
   gccucagcac guaccucua
   19
<210> 2
   <211> 19
   <212> RNA
   <213> Künstliche Sequenz
<220>
   <223> Beschreibung der künstlichen Sequenz: Oligonucleotid
<400> 2
   uagagguacg ugcugaggc
   19
<210> 3
   <211> 19
   <212> RNA
   <213> Künstliche Sequenz
<220>
   <223> Beschreibung der künstlichen Sequenz: Oligonucleotid
<400> 3
   cccuugauga uauguauuu
   19
<210> 4
   <211> 19
   <212> RNA
   <213> Künstliche Sequenz
<220>
   <223> Beschreibung der künstlichen Sequenz: Oligonucleotid
<400> 4
   aaauacauau caucaaggg
   19
<210> 5
   <211> 19
   <212> RNA
   <213> Künstliche Sequenz
<220>
   <223> Beschreibung der künstlichen Sequenz: Oligonucleotid
<400> 5
   gccagcuuau acacaagaa
   19
<210> 6
   <211> 19
   <212> RNA
   <213> Künstliche Sequenz
<220>
   <223> Beschreibung der künstlichen Sequenz: Oligonucleotid
<400> 6
   uucuugugua uaagcuggc
   19
<210> 7
   <211> 19
   <212> RNA
   <213> Künstliche Sequenz
<220>
   <223> Beschreibung der künstlichen Sequenz: Oligonucleotid
<400> 7
   gaucaagaaa uacagacua
   19
<210> 8
   <211> 19
   <212> RNA
   <213> Künstliche Sequenz
<220>
   <223> Beschreibung der künstlichen Sequenz: Oligonucleotid
<400> 8
   uagucuguau uucuugauc
   19
<210> 9
   <211> 19
   <212> RNA
   <213> Künstliche Sequenz
<220>
   <223> Beschreibung der künstlichen Sequenz: Oligonucleotid
<400> 9
   caagaaauac agacuacaa
   19
<210> 10
   <211> 19
   <212> RNA
   <213> Künstliche Sequenz
<220>
   <223> Beschreibung der künstlichen Sequenz: Oligonucleotid
<400> 10
   uuguagucug uauuucuug
   19
<210> 11
   <211> 19
   <212> RNA
   <213> Künstliche Sequenz
<220>
   <223> Beschreibung der künstlichen Sequenz: Oligonucleotid
<400> 11
   agacuacaac aggcccaaa
   19
<210> 12
   <211> 19
   <212> RNA
   <213> Künstliche Sequenz
<220>
   <223> Beschreibung der künstlichen Sequenz: Oligonucleotid
<400> 12
   uuugggccug uuguagucu
   19
<210> 13
   <211> 19
   <212> RNA
   <213> Künstliche Sequenz
<220>
   <223> Beschreibung der künstlichen Sequenz: Oligonucleotid
<400> 13
   gucagauaca acagcauuu
   19
<210> 14
   <211> 19
   <212> RNA
   <213> Künstliche Sequenz
<220>
   <223> Beschreibung der künstlichen Sequenz: Oligonucleotid
<400> 14
   aaaugcuguu guaucugac
   19
<210> 15
   <211> 19
   <212> RNA
   <213> Künstliche Sequenz
<220>
   <223> Beschreibung der künstlichen Sequenz: Oligonucleotid
<400> 15
   gauagauagu ccacugaau
   19
<210> 16
   <211> 19
   <212> RNA
   <213> Künstliche Sequenz
<220>
   <223> Beschreibung der künstlichen Sequenz: Oligonucleotid
<400> 16
   auucagugga cuaucuauc 19
<210> 17
   <211> 19
   <212> RNA
   <213> Künstliche Sequenz
<220>
   <223> Beschreibung der künstlichen Sequenz: Oligonucleotid
<400> 17
   ggauacggau augaaaucu 19
<210> 18
   <211> 19
   <212> RNA
   <213> Künstliche Sequenz
<220>
   <223> Beschreibung der künstlichen Sequenz: Oligonucleotid
<400> 18
   agauuucaua uccguaucc 19
<210> 19
   <211> 19
   <212> RNA
   <213> Künstliche Sequenz
<220>
   <223> Beschreibung der künstlichen Sequenz: Oligonucleotid
<400> 19
   guacgcaaac acuuuaugu 19
<210> 20
   <211> 19
   <212> RNA
   <213> Künstliche Sequenz
<220>
   <223> Beschreibung der künstlichen Sequenz: Oligonucleotid
<400> 20
   acauaaagug uuugcguac
   19
<210> 21
   <211> 19
   <212> RNA
   <213> Künstliche Sequenz
<220>
   <223> Beschreibung der künstlichen Sequenz: Oligonucleotid
<400> 21
   aaugcaacuc ucaccuuaa
   19
<210> 22
   <211> 19
   <212> RNA
   <213> Künstliche Sequenz
<220>
   <223> Beschreibung der künstlichen Sequenz: Oligonucleotid
<400> 22
   uuaaggugag aguugcauu
   19
<210> 23
   <211> 19
   <212> RNA
   <213> Künstliche Sequenz
<220>
   <223> Beschreibung der künstlichen Sequenz: Oligonucleotid
<400> 23
   gaccaucaau aauuacacu
   19
<210> 24
   <211> 19
   <212> RNA
   <213> Künstliche Sequenz
<220>
   <223> Beschreibung der künstlichen Sequenz: Oligonucleotid
<400> 24
   aguguaauua uugaugguc
   19
<210> 25
   <211> 19
   <212> RNA
   <213> Künstliche Sequenz
<220>
   <223> Beschreibung der künstlichen Sequenz: Oligonucleotid
<400> 25
   acguguaaag cugugacau
   19
<210> 26
   <211> 19
   <212> RNA
   <213> Künstliche Sequenz
<220>
   <223> Beschreibung der künstlichen Sequenz: Oligonucleotid
<400> 26
   augucacagc uuuacacgu
   19
<210> 27
   <211> 21
   <212> RNA
   <213> Künstliche Sequenz
<220>
   <223> Beschreibung der künstlichen Sequenz: Oligonucleotid
<400> 27
   uuaaagagag uggagccugg u
   21
<210> 28
   <211> 21
   <212> RNA
   <213> Künstliche Sequenz
<220>
   <223> Beschreibung der künstlichen Sequenz: Oligonucleotid
<400> 28
   accaggcucc acucucuuua a 21
<210> 29
   <211> 19
   <212> RNA
   <213> Künstliche Sequenz
<220>
   <223> Beschreibung der künstlichen Sequenz: Oligonucleotid
<400> 29
   ggaggauacg gauaugaaa 19
<210> 30
   <211> 19
   <212> RNA
   <213> Künstliche Sequenz
<220>
   <223> Beschreibung der künstlichen Sequenz: Oligonucleotid
<400> 30
   uuucauaucc guauccucc 19
<210> 31
   <211> 19
   <212> RNA
   <213> Künstliche Sequenz
<220>
   <223> Beschreibung der künstlichen Sequenz: Oligonucleotid
<400> 31
   gagcuaaauu acacauuga 19
<210> 32
   <211> 19
   <212> RNA
   <213> Künstliche Sequenz
<220>
   <223> Beschreibung der künstlichen Sequenz: Oligonucleotid
<400> 32
   ucaaugugua auuuagcuc
   19
<210> 33
   <211> 19
   <212> RNA
   <213> Künstliche Sequenz
<220>
   <223> Beschreibung der künstlichen Sequenz: Oligonucleotid
<400> 33
   caucuacgcu gacaaugaa
   19
<210> 34
   <211> 19
   <212> RNA
   <213> Künstliche Sequenz
<220>
   <223> Beschreibung der künstlichen Sequenz: Oligonucleotid
<400> 34
   uucauuguca gcguagaug
   19
<210> 35
   <211> 19
   <212> RNA
   <213> Künstliche Sequenz
<220>
   <223> Beschreibung der künstlichen Sequenz: Oligonucleotid
<400> 35
   cucuauauuu agauuguua
   19
<210> 36
   <211> 19
   <212> RNA
   <213> Künstliche Sequenz
<220>
   <223> Beschreibung der künstlichen Sequenz: Oligonucleotid
<400> 36
   uaacaaucua aauauagag
   19
<210> 37
   <211> 19
   <212> RNA
   <213> Künstliche Sequenz
<220>
   <223> Beschreibung der künstlichen Sequenz: Oligonucleotid
<400> 37
   gguugaaugc accacucaa
   19
<210> 38
   <211> 19
   <212> RNA
   <213> Künstliche Sequenz
<220>
   <223> Beschreibung der künstlichen Sequenz: Oligonucleotid
<400> 38
   uugaguggug cauucaacc
   19
<210> 39
   <211> 19
   <212> RNA
   <213> Künstliche Sequenz
<220>
   <223> Beschreibung der künstlichen Sequenz: Oligonucleotid
<400> 39
   ugguagaauu ggagaguaa
   19
<210> 40
   <211> 19
   <212> RNA
   <213> Künstliche Sequenz
<220>
   <223> Beschreibung der künstlichen Sequenz: Oligonucleotid
<400> 40
   uuacucucca auucuacca
   19
<210> 41
   <211> 19
   <212> RNA
   <213> Künstliche Sequenz
<220>
   <223> Beschreibung der künstlichen Sequenz: Oligonucleotid
<400> 41
   cagugugguu uguguuuga
   19
<210> 42
   <211> 19
   <212> RNA
   <213> Künstliche Sequenz
<220>
   <223> Beschreibung der künstlichen Sequenz: Oligonucleotid
<400> 42
   ucaaacacaa accacacug
   19
<210> 43
   <211> 19
   <212> RNA
   <213> Künstliche Sequenz
<220>
   <223> Beschreibung der künstlichen Sequenz: Oligonucleotid
<400> 43
   cggaagcuau gacuuauga
   19
<210> 44
   <211> 19
   <212> RNA
   <213> Künstliche Sequenz
<220>
   <223> Beschreibung der künstlichen Sequenz: Oligonucleotid
<400> 44
   ucauaaguca uagcuuccg
   19
<210> 45
   <211> 19
   <212> RNA
   <213> Künstliche Sequenz
<220>
   <223> Beschreibung der künstlichen Sequenz: Oligonucleotid
<400> 45
   uucuccgaac gugucacgu
   19
<210> 46
   <211> 19
   <212> RNA
   <213> Künstliche Sequenz
<220>
   <223> Beschreibung der künstlichen Sequenz: Oligonucleotid
<400> 46
   acgugacacg uucggagaa
   19